# EUROPEAN PATENT APPLICATION

(11) **EP 1 402 896 A1**
(43) Date of publication of application: **31.03.2004**
(21) Application number: 03026065.7
(22) Date of filing: 14.07.2000
(51) Int. Cl.: A61K 35/78, A61K 38/13, A61P 13/12

(54) **Green tea extract and use thereof in treating renal dysfunction**

(62) Divisional of application: 00115330.3
(71) Applicant: HEALTHECON AG, 4051 Basel (CH)
(72) Inventor: Schneider, Heinz, 1792 Cordast (CH)
(74) Representative: Grünecker, Kinkeldey, Stockmair & Schwanhäusser Anwaltssozietät

(57) **Abstract**

A method for the use of green tea extract in water or oil soluble form in the preparation of a medicament or nutritional formulation for the prophylaxis and/or therapy of renal dysfunction induced by cyclosporins or ascomycins.

## Description

The present invention relates to pharmaceutical compositions or nutritional formulations comprising a water or oil soluble green tea extract and a cyclosporin, ascomycin and/or at least one amino acid and the use of a water or oil soluble green tea extract in the preparation of a pharmaceutical composition or nutritional formulation for the prophylactic and/or therapeutic treatment of renal dysfunction, which may be induced by substances belonging to the classes of cyclosporins and/or ascomycins.

Cyclosporins comprise a class of structurally distinct, cyclic, poly-N-methylated endecapeptides, generally possessing immunosuppressive, anti-inflammatory, antiviral and/or anti-parasitic activity, each to a greater or lesser degree. The first of the cyclosporins to be identified was the fungal metabolite Cyclosporin A, or Ciclosporin, and its structure is given in The Merck Index, 11^{th} Edition; Meck & Co., Inc.; Rahway, New Jersey, USA (1989) under listing 2759. Later cyclosporins to be identified are cyclosporins B, C, D and G which are also listed in The Merck Index under listing 2759. A large number of synthetic analogues are also known and representative examples are disclosed in EP 296 123, EP 484 281, and GB 2222770. Cyclosporin A and its structurally similar analogues and derivatives as well as metabolites thereof are generally referred to as "cyclosporins" for the purposes of this specification.

Ascomycins, of which FK-506 is the best known, are another class of generally immunosuppressive substances, also referred to as macrolide immunosuppressants. FK-506 is a macrolide immunosuppressant that is produced by **Streptomyces tsukubaensis No. 9993.** The structure of FK-506 is given in the appendix to The Merck Index, supra, as item A5. A large number of related compounds which retain the basic structure and immunological properties of FK-506 are also known. These compounds are described in various publications, for example, EP 184162, EP 315973, EP 323042, EP 423714, EP 427680, EP 465426, EP 474126, WO 91/13889, WO 91/19495, EP 484935, EP 532088, EP 532089, WO 93/5059 and the like. Ascomycin, FK-506 and their structurally similar analogues and derivatives as well as metabolites thereof are termed collectively "ascomycins" in this specification.

Due to their extremely useful pharmaceutical properties, cyclosporins (Cyclosporins A and G in particular) and ascomycins (e.g. FK-506) have wide application in, for example, the prevention of transplant rejection, in the treatment of auto-immune diseases such as rheumatoid arthritis and psoriasis, and also in the treatment of multidrug-resistance. Cyclosporins and ascomycins also have certain side effects, the most notable being renal dysfunction, in particular nephrotoxicity, especially at higher doses. Nephrotoxicity is characterized by diminished renal blood flow and glomerular filtration with corresponding elevations in serum creatinine, alkaline phosphatase and urea as well as proximal cell swelling and necrosis and infiltration of macrophages. In some studies, hypoxia has been shown to damage proximal tubules rather selectively, and a decrease in blood flow could lead to hypoxia. Evidence has been presented implicating intracellular calcium in this pathology, and calcium channel blockers are effective in minimizing injury. However, Ca channel blockers cannot be given without impunity. Accordingly, there is currently no known useful therapy for this important side effect on the kidney.

Therefore, the problem underlying the invention is to provide a pharmaceutical composition or nutritional formulation for the prophylactic and/or therapeutic treatment of renal dysfunction, which may be induced by cyclosporins or ascomycins.

The solution of this problem is a pharmaceutical composition or nutritional formulation comprising
a water or oil soluble green tea extract, and
a prophylactically and/or therapeutically effective amount of at least one cyclosporin or at least one ascomycin.

Another solution of this problem is a pharmaceutical composition or nutritional formulation comprising
a water or oil soluble green tea extract, and
at least one amino acid selected from the group consisting of glycine, alanine, serine, L-arginine, a physiologically acceptable compound associated with the synthesis of nitric oxide, physiologically acceptable salts thereof and mixtures thereof.

The water or oil soluble green tea extract will, in the following, be referred to as "green tea extract".

The green tea extract used according to the invention preferably comprises green teas nutritional components such as amino acids, polyphenols, vitamins, fluorides, saccharides, minerals and caffeine. The amino acid may be theanine and the polyphenols may preferably be selected from the group consisting of six derivatives of Catechin, namely (-)-Epigallocatechin Gallate (EGCg), (-)-Epigallicatechin (EGC), (-)-Epicatechin Gallate (ECg), (+)-Gallocatechin (GC), (-)-Epicatechin (EC) and (+)-Catechin (C) and mixtures thereof. Commercially available green tea extracts which may be used according to the present invention are Ryokucha GT-1, Sunflavon P, Sunphenon 100S, Sonphenon DCF-1, Suntheanine, and Sunkatol, all available from Taiyo Kagaku company.

The green tea extract to be used according to the present invention may be obtained by steeping green tea leaves into ten weights of hot water at 95°C for 30 minutes with gentle stirring. The obtained mixture is pressed, the juice filtered and the filtrate is concentrated to above a quarter by volume in vacuo. The water extract is partitioned with an equal volume of ethylacetate three times, and the ethylacetate layer is brought to dryness in vacuo and thereby a green tea extract is obtained.

For use in the pharmaceutical compositions and nutritional formulations, green tea extract is conveniently employed in dried or liquid, oily form. Green tea extract may also be used in the form of concentrated solutions according to the invention. If the green tea extract is used in nutritional formulations it is preferred to use a water soluble green tea extract.

In a first embodiment of the invention, the pharmaceutical composition or nutritional formulation comprises a water or oil soluble green tea extract and at least one cyclosporin and/or at least one ascomycin.

The green tea extract is preferably present in the pharmaceutical compositions or nutritional formulations of the present invention in an amount of about 0.001 to about 20 grams, preferably about 0.01 to 10 grams per 100 grams of the pharmaceutical composition or nutritional formulation.

The cyclosporins or ascomycins present in the pharmaceutical compositions or nutritional formulations of the present invention may be selected from the group consisting of cyclosporin A, B, C, G, PSC 833 ([3'-desoxy-3'-oxo-MeBmt]-1-(Val]-2-cyclosporin, FK-506, ASM (33-epi-chloro-33-desoxy-ascomycin) or combinations thereof, most preferably cyclosporin A, G, FK-506, immunosuppressive cyclosporins or ascomycins, and combinations thereof.

The cyclosporin may be present in the pharmaceutical compositions or nutritional formulations of the present invention in an amount of about 5 to 200 mg, preferably about 10 to 100 mg per dose unit, such as per soft gel capsule or per ml of drinking solution.

The ascomycin, in particular FK 506, may be present in the pharmaceutical compositions or nutritional formulations of the present invention in an amount of about 0.1 to 50 mg, preferably about 0.5 to 5 mg per dose unit, such as per soft gel capsule or per ml of drinking solution.

In a second embodiment of the invention the pharmaceutical composition or nutritional formulation, comprises a green tea extract and at least one amino acid selected from the group consisting of glycine, alanine, serine, L-arginine, physiologically acceptable compounds associated with the synthesis of nitric oxide, pharmaceutically acceptable salts thereof and mixtures thereof.

The green tea extract may be present in amounts as discussed for the first embodiment above.

The at least one amino acid present in the pharmaceutical compositions or nutritional formulation of the invention is preferably selected from the group consisting of glycine, L-arginine, physiologically acceptable salts thereof and mixtures thereof.

Examples of suitable salts of amino acids for this invention include, but are not limited to, phosphates, citrates, acetates, malates, tartrates, fumarates, lactates, hydrates and the like.

The amino acid or mixtures of amino acids can be present in the pharmaceutical compositions or nutritional formulations of the present invention in an amount of at least about 0.01 wt-%, preferably about 1.5 to 80 wt-%, based on the weight of the pharmaceutical compositions or nutritional formulations.

If at least one amino acid and L-arginine and/or other physiologically acceptable compound associated with the synthesis of nitric oxide is present, the weight ratio of the at least one amino acid to L-arginine and/or the other physiologically acceptable compound associated with the synthesis of nitric oxide is preferably from 1:2 to 4:1.

The pharmaceutical compositions or nutritional formulations according to the second embodiment may additionally comprise at least one cyclosporin and/or at least one ascomycin, preferably cyclosporin A and/or ascomycin FK 506, in amounts as described above for the first embodiment of the invention.

The pharmaceutical compositions of nutritional formulation of the invention may be administered to the patient orally, enterally or parenterally.

The oral administration route is preferred, particularly for subsequent or prophylactic treatment. Typical pharmacologically acceptable formulation forms for oral administration will further comprise pharmacologically acceptable diluents, carriers, microemulsions, vitamins, pigments and/or other adjuvants well known to the skilled person to be suitable for incorporation into such formulations and optionally a cyclosporin or a macrolide immunosuppressive drug.

The pharmaceutical compositions or nutritional formulation is conveniently administered in the form of an aqueous drinking solution using ethanol and edible oils as carrier for the hydrophobic ciclosporins or ascomycins or in the form of galenic formulations for oral administration. The pharmaceutical compositions or nutritional formulations in a form suitable for oral application is accordingly preferably in aqueous or in soft gelatine capsule form.

The pharmaceutical compositions or nutritional formulations of the invention may be so formulated as to deliver to the patient from about 0.01 to about 20g, preferably about 0.1 to about 15g, particularly preferred about 0.25 to about 10g of green tea extract of the invention per 24 hours, corresponding to a daily dose of 5X100mg/kg body weight (BW range 50-100kg). The amount of pharmaceutical compositions or nutritional formulations to be administered depends to a large extent on the patients' specific requirements. Such daily amounts of green tea extract are suitable for treatment of the desired effects as well as for prophylactic pretreatment.

The pharmaceutical compositions or nutritional formulations of the invention are useful in the prophylactic and/or therapeutic treatment of renal dysfunction. In particular the pharmaceutical compositions or nutritional formulations of the present invention are effective in inhibiting and/or ameliorate renal dysfunction induced by cyclosporin or ascomycin, particularly by cyclosporin A, FK-506, and PSC 833. Preferably the pharmaceutical compositions and nutritional formulations of the invention are used in the prophylactic and/or therapeutic treatment of the renal dysfunction nephrotoxicity.

The pharmaceutical compositions and nutritional formulations of the invention can be administered to the patient before, during and/or after the occurrence of a renal dysfunction.

Where nutritional formulation of the invention is intended for use as a dietary supplement, the amount of energy supplied by it should not be too excessive, in order not to unnecessarily suppress the patients' appetite. The supplement should conveniently comprise energy sources, predominantly or exclusively carbohydrates and amino acids, in an amount supplying from 5 to 500 Kcal/day, preferably 10 to 250 Kcal/day. The contribution of the carbohydrate source, nitrogen source and lipid source to the total daily caloric may vary within wide ranges. For use as a dietary supplement, the administration may be in powder or liquid form.

The treatment period will coincide with the treatment with the immunosuppressive drug.

The supplement will conveniently be administered in the form of unit doses suitable for administration of the supplement 1 to 4 times per day. Where the nutritional supplements of the invention comprise energy sources, it is appropriate not to supply more than 500 Kcal/day. Apart from this limitation with respect to the energy supply, dietary supplements of the invention for preventing and/or treating nephrotoxicity induced by cyclosporins or macrolide immunosuppressive drugs can and will conveniently be supplied in the form of drinking solutions.

The pharmaceutical compositions of nutritional formulations of the invention may be obtained in a manner known per se, e.g. by admixing the ingredients.

A third embodiment of the present invention is the use of a green tea extract for the preparation of a pharmaceutical composition or nutritional formulation for the prophylactic and/or therapeutic treatment of renal dysfunction.

The amount of green tea extract to be used is as described above for the pharmaceutical compositions and nutritional formulations of the invention.

It has been found that green tea extract inhibits and/or ameliorates one of the major side effects of chronic cyclosporin A and FK-506 administration - nephrotoxicity. Green tea extract prevents the decrease in glomerular filtration rate. Green tea extract also prevents the elevation in serum creatinine and urea, and tends to minimize the elevation in alkaline phosphatase due to cyclosporin A and FK-506. The swelling and necrosis of proximal tubules and macrophage infiltration are also all prevented by green tea extract in water and oil soluble form. Thus, it is clear that green tea extract has a major protective action on nephrotoxicity due to cyclosporin A or FK-506.

The invention is further illustrated by the following Examples referred to Fig. 1 to 6 which are not intended in any way to limit the scope of the claimed invention.

Fig. 1 shows effects of Cyclosporin A (CsA) and water-soluble green tea extract on body weight changes

Fig. 2 shows effects of Cyclosporin A (CsA) and water-soluble green tea extract (GTE) on glomerular filtration rates

Fig. 3 shows dose-dependency of water-soluble green tea extract's effect on glomerular filtration rates

Fig. 4 shows-effects of Cyclosporin A (CsA) and oil soluble green tea extract (OSGTE) on glomerular filtration rates

Fig. 5 shows effects of Cyclosporin A (CsA) and green tea extract (GTE) on histological changes in the kidney

Fig. 6 shows effects of FK-506 and green tea extract (GTE) on glomerular filtration rates.

### EXAMPLE 1

### Prevention of Cyclosporin A induced nephrotoxicity by green tea extract

### Reagents

Cyclosporin A (Sandimmune oral solution) was the Norvatis product (Basel, Switzerland). Water soluble green tea extract (Sunphenon) and oil (soybean oil) soluble green tea extract (Sunkatol) were formed from Taiyo Kagaku Co. (Yokkaichi, Mie, Japan). Creatinine analytic kit and a-(4-pyridyl I-oxide)-N-tert-butylnitrone (4-POBN) were obtained from Sigma Chemical Co., St. Louis, MO.

### Animals and Treatment

Male Sprague-Dawley rats (200-250 g) were fed a semi-synthetic powdered diet (AIN 76) containing 0-0.1% water-soluble green tea extract (GTE) for 3 days prior to cyclosporin A. Oil soluble green tea extract (OSGTE) was added to cyclosporin A/olive oil solution at concentrations of 0-2.8% and given to some rats by gavage daily. Previous studies showed that cyclosporin A at doses ranging from 25-50 mg/kg caused typical nephrotoxicity including reduced glomerular filtration rates, increased serum creatinine and pathological changes. Accordingly, in this study, cyclosporin A (25 mg/kg dissolved in olive oil at a concentration of 10 mg/m) or an equivalent volume of olive oil vehicle was given by oral gavage. Diet consumption was 8.2 g/100g body weight/day in rats studied. All animals were given humane care in compliance with institutional guidelines.

### Glomerular Filtration Rates and Serum Creatinine

Animals were placed in metabolic cages and urine was collected daily. Creatinine levels in urine and sera were determined using commercially available kits, and glomerular filtration rates were calculated from the ratio of creatinine in the urine/blood the volume of urine produced in 24 hours and the body weight.

### Histology

On the day of sacrifice, animals were anesthetized with pentobarbital (50 mg/kg, i.p.), the abdomen was opened and the aorta was cannulated with a 24 gauge i.v. gannula with the tip placed near the renal arterial branches. The aorta was clamped above and ligated beneath the renal branches and the left kidney was rinsed with 5 ml normal saline and perfusion-fixed with 10% formaldehyde solution in phosphate buffered saline. The kidney was removed, cut in 0.5 mm thick slices and placed in 10% formalin for 48 hours. Sections were stained with hematoxylin-eosin and analyzed microscopically.

### Statistical Analysis

All groups were compared using ANOVA - Student-Newman-Keul's post-hoc test as appropriate. Differences were considered significant at the p<0.05 level.

### Effects of Cyclosporin A and Green Tea Extracts on Body Weight and Renal Function

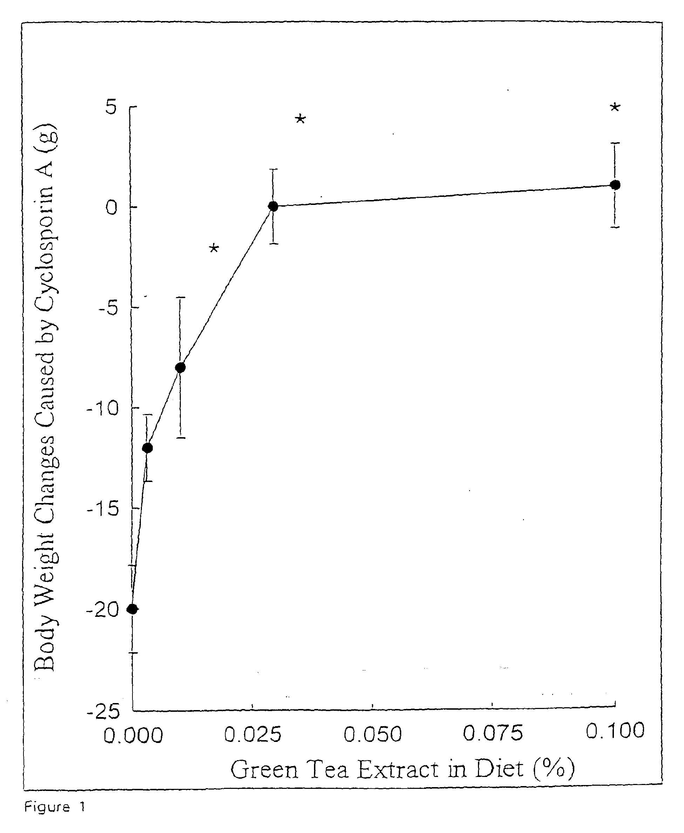

Rats fed 0-0.1% based on the diet of water-soluble green tea extract gained similar body weights during days of prefeeding period. One day after cyclosporin A treatment, rats fed control diets lost about 20 g of body weight. Water soluble green tea extract (0.003-0.1%) blunted the decreases in a dose-dependent manner (Fig. 1), indicating that green tea extracts prevent body weight loss caused by cyclosporin A.

Rats were fed AIN 76 powdered diet containing 0-0.1% water-soluble green tea extract (GTE) as described in the "Methods". Cyclosporin A (25 mg/kg) was given by gavage daily to all rats. Fig. 1 depicts body weight changes 24 hours after the first dose of cyclosporin A. Values are means ± S.E.M. (ANOVA; n = 5-6 in each group). *, p <0.05 compared to the rats received control diet by Student-Newman-Keul's post-hoc test.

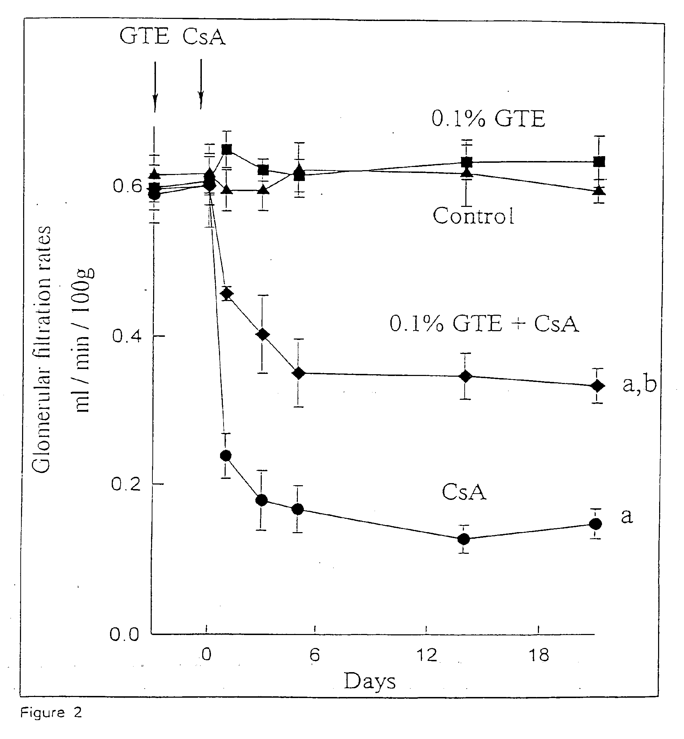

When rats were fed a semi-synthetic powdered diet containing 0% (control) or 0.1% watersoluble green tea extract starting 3 days prior to cyclosporin A (25 mg/kg, i.g. for 3 weeks), urine samples were collected using metabolic cages, and glomerular filtration rates were calculated from the ratio of creatinine in the urine/blood, the volume of urine produced in 24 hours, and the body weight as shown in Fig. 2. Values are means ± S.E.M. (ANOVA; n = 5-6 in each group). a, p<0.05 compared to control; b, p<0.05 compared to the CsA group by Student-Newman-Keul's post-hoc test.

Glomerular filtration rates, indicators of renal function, was around 0.6 ml/min/100g body weight in controls and was not altered by water-soluble green tea extract (Fig. 2). Glomerular filtration rate declined gradually with time of cyclosporin A treatment. After 5 days of treatment glomerular filtration rates were decreased by about 70% (Fig. 2) as expected. In rats fed 0.1 % green tea extract, decreases in glomerular filtration rate was half as large as in rats receiving control diets (Fig. 2).

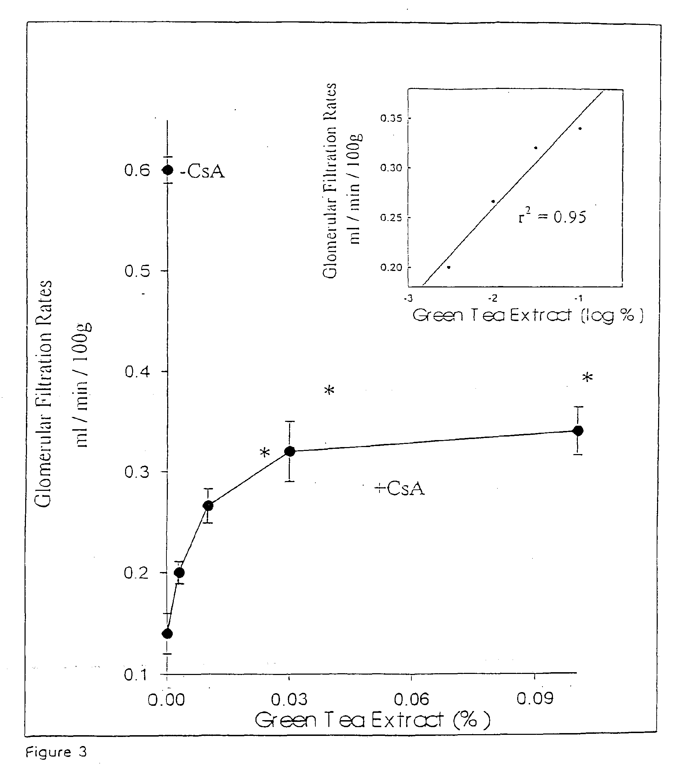

When rats were fed a semi-synthetic powdered diet containing 0, 0.003, 0.01, 0.03 and 0.1 % water-soluble green tea extract (GTE) under the same conditions as shown in Fig 2 and all rats received cyclosporin A treatment for 3 weeks, glomerular filtration rates blunted decreases in glomerular filtration rates caused by cyclosporin A in a dose-dependent manner (Fig. 3). Values are means ± S.E.M. (ANOVA; n = 5-6 in each group) and "*" indicates p<0.05 compared to control diet by Student-Newman-Keul's post-hoc test.

Dietary water-soluble green tea extract (0.003 - 0.1 %) blunted decreases in glomerular filtration rates caused by cyclosporin A in a dose-dependent manner (Fig. 3).

The percentage of green tea extract in the diet which caused half-maximal effect was about 0,008% (Fig. 3, insert).

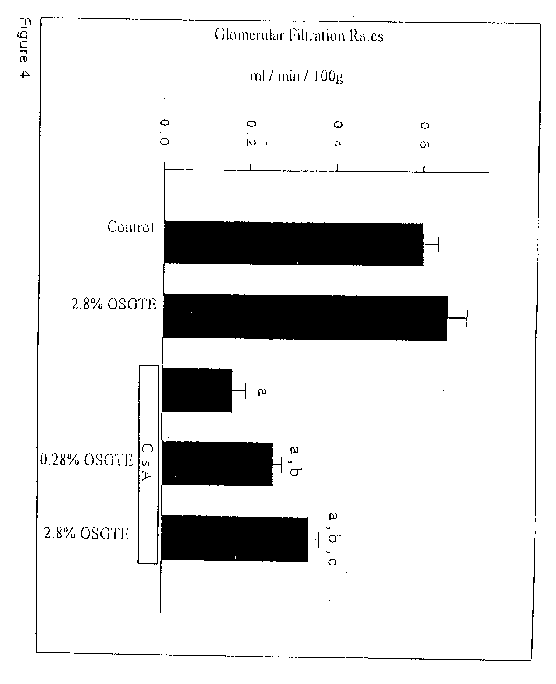

The fat-soluble green tea extract also minimized the inhibition on glomerular filtration rates caused by cyclosporin A in a dose-dependent manner (Fig. 4).

As shown in Fig. 4, rats were gavaged daily with oil soluble green tea extract (0-2.8%) starting 3 days prior to cyclosporin A treatment (25 mg/kg, i.g. for 3 weeks). An equal volume of soybean oil vehicle was given to the control group. Urine samples were collected using metabolic cages, and glomerular filtration rates were calculated from the ratio of creatinine in the urine/blood, the volume of urine produced in 24 hours, and the body weight. Values are means ± S.E.M. (ANOVA; n=5-6 in each group); a, p<0.05 compared to the control group by Student-Newman-Keul's post-hoc test; b, p<0.05 compared to the CsA group by Student-Newman-Keul's post-hoc test; c, p<0.05 compared to 0.28% OSGTE group by Student-Newman-Keul's post-hoc test.

In rats treated with 2.8% Sunkatol, decreases in glomerular filtration rates induced by cyclosporin A was blunted by -45% (Fig. 4).

### Effects of Cyclosproin A and Green Tea Extracts on Renal Histology

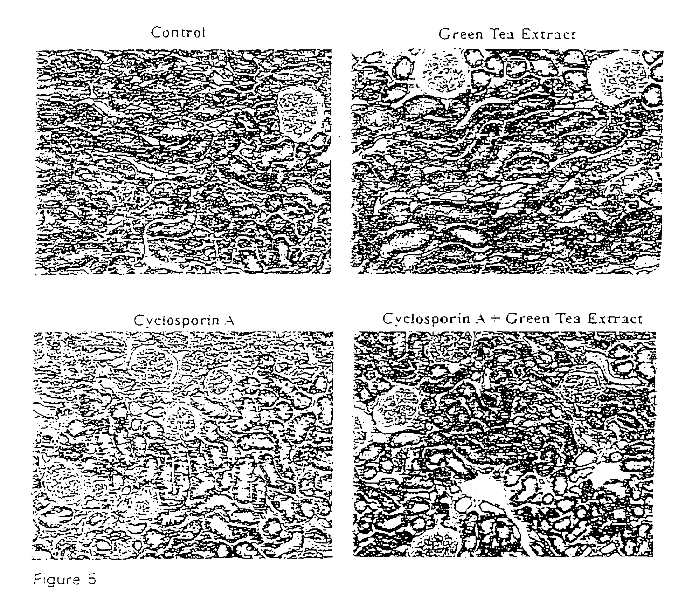

In Figure 5, the histology of kidneys from this study is shown.

Rats were fed a semi-synthetic powdered diet containing 0% (control) or 0.1 % watersoluble green tea extract (GTE) starting 3 days prior to cyclosporin A treatment (25 mg/kg, i.g. for 3 weeks), Sections of the kidney were stained with hematoxylin and eosin and examined microscopically - original magnification was 100x. Typical sections from at least 4 animals in each group. A, control; B, Sunphenon; C. CsA - treated group; and D, CsA - GTE.

In the upper panel (A) normal renal cortex from a rat on the control diet is depicted. Water soluble green tea extract: (0.1 %) feeding did not cause histological changes (B). Cyclosporin A (C) caused loss of brush borders and swelling of the proximal tubules, necrosis, degeneration and white blood cell infiltration, as has been reported previously. These effects were minimized by 0.1 % green tea extract - containing diet (D).

### EXAMPLE 2

### Prevention of FK 506 - induced nephrotoxicity by green tea extract

### Reagents

FK-506 (Tacrolimus i.v. solution) was from Fujisawa Ireland, Ltd. (Killorglin, Ireland). Water soluble green tea extract (Sunphenon) and oil (soybean oil) soluble green tea extract (Sunkatol) were from Taiyo Kagaku Co. (Yokkaichi, Mie, Japan). Creatinine analytic kit and a-(4-pyridyl I -oxide)-N-tert-butylnitrone (4-POBN) were obtained from Sigma Chemical Co., St. Louis, MO.

### Animals and Treatment

Male Sprague-Dawley rats (200-250g) were fed a semi-synthetic powdered diet (AIN76) containing 0-0.1% water-soluble green tea extract (GTE) for 3 days prior to FK-506 treatment. Oil soluble green tea extract (OSGTE) was given by gavage daily. Previous studies showed that FK-506 ranging from 3-10 mg/kg caused typical nephrotoxicity including reduced glomerular filtration rates, increased serum creatinine and pathological changes. Accordingly, in this study, FK-506 (3 mg/kg, i.p.) was dissolved in normal saline and injected daily for 21 days. Diet consumption was 8.2 g/100g body weight/day in rats studied. All animals were given humane care in compliance with institutional guidelines.

### Glomerular Filtration Rates and Serum Creatinine

Animals were placed in metabolic cages and urine was collected daily. Creatinine levels in urine and sera were determined using commercially available kits, and glomerular filtration rates were calculated from the ratio of creatinine in the urine/blood, the volume of urine produced in 24 hours, and the body weight.

### Statistical Analysis

All groups were compared using ANOVA - Student-Newman-Keul's post-hoc test as appropriate. Differences were considered significant at the p<0.05 level.

### Results

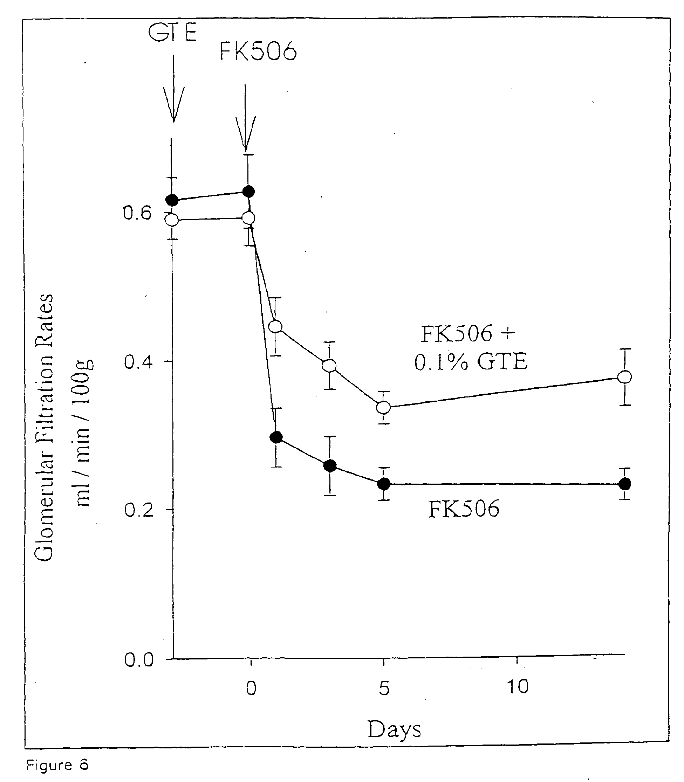

### Effects of FK-506 and Green Tea Extracts on Renal Function (Fig. 6)

Rats were fed daily with a semi-synthetic powdered diet containing 0% (control) or 0.1 % water-soluble green tea extract (GTE) starting 3 days prior to FK-506 treatment (3 mg/kg, i.p. for 5 days). Urine Samples were collected using metabolic cages and glomerular filtration rates were calculated from the ratio of creatinine in the urine/blood, the volume of urine produced in 24 hours, and the body weight. Values are means ± S.E.M. (ANOVA; n=5-6 in each group), p<0.05 compared to the FK506 group by Student-Newman-Keul's post-hoc test.

Glomerular filtration rates, indicators of renal function, was around 0.6 ml/min/100 g body weight in controls and was not altered by oil soluble green tea extract (Fig. 6). Glomerular filtration rate declined gradually with time under FK-506 treatment. After 5 days of treatment, glomerular filtration rates were decreased by about 70% as expected. Dietary oil soluble green tea extract (0.28 - 2.8%) blunted decreases in glomerular filtration rates caused by FK- 506 in a dose-dependent manner (Fig 6).

## Claims

1. Pharmaceutical composition or nutritional formulation comprising:
a water or oil soluble green tea extract, and at least one amino acid selected from the group consisting of glycine, alanine, serine, L-arginine, a physiologically acceptable compound associated with the synthesis of nitric oxide,
physiologically acceptable salts thereof and mixtures thereof.

2. Pharmaceutical composition or nutritional formulation according to claim 1, wherein the amino acid is glycine or a physiologically acceptable salt thereof.

3. Pharmaceutical composition or nutritional formulation according to claims 1 or 2, wherein L-arginine and/or a physiologically acceptable compound associated with the synthesis of nitric oxide is present in admixture with at least one further amino acid.

4. Pharmaceutical composition or nutritional formulation according to claim 3, wherein the weight ratio of L-arginine and/or a physiologically acceptable compound associated with the synthesis of nitric oxide to the further amino acid is from 2:1 to 1:4.

5. Pharmaceutical composition or nutritional formulation according to claims 1 to 4, additionally comprising at least one cyclosporin and/or at least one ascomycin.

6. Pharmaceutical composition or nutritional formulation according to any one of the preceding claims, wherein the water or oil soluble green tea extract is present in an amount of about 0.01 to about 20g per 100 g of the pharmaceutical composition or nutritional formulation.

7. Use of a pharmaceutical composition or nutritional formulation according to claims 1 to 6 for the preparation of a medicament for the prophylactic and/or therapeutic treatment of renal dysfunction.

8. Use according to claim 7 wherein the renal dysfunction is induced by cyclosporin and/or ascomycin.

9. Use according to claims 7 or 8, wherein the renal dysfunction is nephrotoxicity.
